(19) Europäisches Patentamt / European Patent Office / Office européen des brevets

(11) **EP 2 794 006 B1**

(12) **EUROPÄISCHE PATENTSCHRIFT**

(45) Veröffentlichungstag und Bekanntmachung des Hinweises auf die Patenterteilung:
**29.03.2017 Patentblatt 2017/13**

(21) Anmeldenummer: **12815651.0**

(22) Anmeldetag: **03.12.2012**

(51) Int Cl.:
***A61N 5/10*** *(2006.01)*

(86) Internationale Anmeldenummer:
**PCT/EP2012/074223**

(87) Internationale Veröffentlichungsnummer:
**WO 2013/092181 (27.06.2013 Gazette 2013/26)**

(54) **VERFAHREN ZUR ERMITTLUNG EINES DOSISEINTRAGS IN EIN ZU BESTRAHLENDES OBJEKT**

METHOD FOR DETERMINING A DOSE ENTERING AN OBJECT THAT IS TO BE IRRADIATED

PROCÉDÉ PERMETTANT DE DÉTERMINER UNE DOSE INTRODUITE DANS UN OBJET DEVANT ÊTRE EXPOSÉ À UN RAYONNEMENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.12.2011 DE 102011056882**

(43) Veröffentlichungstag der Anmeldung:
**29.10.2014 Patentblatt 2014/44**

(73) Patentinhaber: **GSI Helmholtzzentrum für Schwerionenforschung GmbH**
**64291 Darmstadt (DE)**

(72) Erfinder:
• **BERT, Christoph**
**91080 Uttenreuth (DE)**

• **LÜCHTENBORG, Robert**
**42285 Wuppertal (DE)**

(56) Entgegenhaltungen:
**WO-A2-2011/153639 JP-A- 2009 045 229**

• **KRAEMER M ET AL: "TREATMENT PLANNING FOR HEAVY-IRON RADIOTHERAPY: PHYSICAL BEAM MODEL AND DOSE OPTIMIZATION", PHYSICS IN MEDICINE AND BIOLOGY, INSTITUTE OF PHYSICS PUBLISHING, BRISTOL GB, Bd. 45, Nr. 11, 1. November 2000 (2000-11-01), Seiten 3299-3317, XP001146537, ISSN: 0031-9155, DOI: 10.1088/0031-9155/45/11/313**

**Beschreibung**

Die Erfindung betrifft ein Verfahren zur Ermittlung des Dosiseintrags in ein mit einem energetischen Teilchenstrahl zu bestrahlendes Objekt während der Applizierung der Bestrahlung, wobei zumindest zeitweise und/oder zumindest teilweise der Dosiseintrag in Volumenbereiche, welche außerhalb des jeweils vom energetischen Teilchenstrahl bestrahlten Zielvolumenbereichs liegen, ermittelt wird. Die Erfindung betrifft weiterhin ein Therapiekontrollsystem mit zumindest einer Kontrolleinrichtung. Darüber hinaus betrifft die Erfindung ein Bestrahlungssystem.

Die Verwendung von Strahlung unterschiedlichster Art und Energie zur Beaufschlagung, Bearbeitung von zu bearbeitenden Werkstücken bzw. der Veränderung von Materialeigenschaften von zu bearbeitenden Werkstücken hat sich zwischenzeitlich im Stand der Technik für unterschiedlichste Anwendungsgebiete durchgesetzt.

Als Strahlungsart kommt dabei nicht nur Photonenstrahlung (also insbesondere Lichtbeaufschlagung, Beaufschlagung mit Röntgenstrahlung, UV-Licht, Infrarotlicht und dergleichen), sondern auch insbesondere Partikelstrahlung in Betracht. Die Partikel können dabei im Wesentlichen beliebig sein (wobei unter Partikeln in diesem Zusammenhang insbesondere Partikel zu verstehen sind, welche eine - wenn auch gegebenenfalls äußerst kleine - Ruhemasse aufweisen). Rein beispielhaft sind Hadronen und Leptonen zu nennen, insbesondere auch Neutrinos, Elektronen, Positronen, Pionen, Mesonen, Protonen, Neutronen, Atomkerne (beispielsweise He-Kerne), Atome bzw. Moleküle sowie Ionen (insbesondere auch Schwerionen wie Sauerstoffionen, Heliumionen, Neonionen oder Kohlenstoffionen).

Dabei ist allen Bestrahlungsarten gemeinsam, dass mit der Strahlung in dem mit der Strahlung beaufschlagten Gegenstand eine bestimmte Energie deponiert wird. Die Art und Weise, wie diese Energie deponiert wird, unterscheidet sich jedoch zum Teil stark. Während beispielsweise bei Photonenstrahlung der Energieverlust in weiten Energiebereichen annähernd exponentiell zur durchdrungenen Materie ist, weisen Teilchenstrahlen, hier insbesondere hadronische Teilchen (speziell Protonen, Ionen und Schwerionen), einen ausgeprägten, so genannten Bragg-Peak auf. Die Teilchen verlieren also beim Durchdringen von Materie zunächst vergleichsweise wenig Energie auf ihrem Weg. Kurz bevor die Teilchen "stecken bleiben" wird der größte Teil der Energie in die mit der Strahlung beaufschlagte Materie abgegeben. Durch diesen Bragg-Peak können nicht nur zweidimensional strukturierte Dosisbeaufschlagungen realisiert werden, sondern insbesondere auch dreidimensional strukturierte Dosisbeaufschlagungen (also unterschiedliche deponierte Strahlungsdosen in unterschiedlichen Tiefen des bestrahlten Objekts).

Nicht nur die Art der verwendeten Strahlung ist mannigfaltig, sondern auch die Art der mit Strahlung beaufschlagten Objekte. Um lediglich einige technische Anwendungsgebiete zu nennen, ist beispielsweise im Zusammenhang mit der Beaufschlagung mit Photonen an Strukturierungsverfahren mit Masken und Materieabtrag bzw. Materieauftrag bei der Herstellung von strukturierten Halbleiterbauteilen (beispielsweise Speicherbausteine, Mikroprozessoren und dergleichen) zu denken. Photonen können auch zum Schneiden und/oder zum Verschweißen von Werkstücken verwendet werden (insbesondere dann, wenn die Photonenstrahlung in Form eines hochenergetischen Laserstrahls vorliegt).

Ein Anwendungsbeispiel für Elektronenstrahlen ist das so genannte Elektronenstrahlschweißen, mit dem beispielsweise zwei metallische Werkstücke miteinander verschweißt werden können. Selbstverständlich sind auch Trenn- bzw. Strukturierungsvorgänge denkbar.

In der Medizin bzw. der Veterinärmedizin findet Strahlung zu therapeutischen Zwecken Verwendung. Bekannt ist beispielsweise die Verwendung von Röntgenstrahlung zur Erstellung von Röntgenbildern (einschließlich dreidimensionaler Bilder durch so genannte CT-Verfahren; CT für Computertomographie). Auch Elektronenstrahlen werden bereits seit einigen Jahrzehnten in der Medizin verwendet, beispielsweise zur Therapie von Krebstumoren. Zwischenzeitlich hat sich auch die Therapie von Tumoren mit Protonen und Ionen (insbesondere Schwerionen) als feste Größe in der Medizin etabliert. Aufgrund des bereits beschriebenen Bragg-Peaks bei Protonen/Ionen/Schwerionen ist es möglich, durch entsprechende Ansteuerung eines Teilchenstrahls (beispielsweise im Rahmen eines Scanning-Vorgangs) ein dreidimensional abgegrenztes und strukturiertes Gebiet (also insbesondere einen Tumor) in einem Patienten gezielt mit Strahlung zu beaufschlagen, während das umliegende Gewebe weitestgehend geschont wird. Zwischenzeitlich sind Genauigkeiten im Millimeter-Bereich realisierbar.

Bei den Scanverfahren wird üblicherweise ein dünner Teilchenstrahl (häufig auch als bleistiftdünner Teilchenstrahl bezeichnet) verwendet, der über geeignete Ablenkmagneten in lateraler Richtung abgelenkt werden kann (x-y-Ebene) und über eine geeignete Energievariation hinsichtlich seiner Eindringtiefe gesteuert werden kann. Durch entsprechende Variationen von Ablenkung und Energie ist es möglich, die unterschiedlichen, mit einer Dosis zu beaufschlagenden Volumenbereiche des zu bestrahlenden Objekts "anzufahren". Eine Bestrahlung erfolgt in der Regel unter Verwendung eines so genannten Bestrahlungsplans (bzw. einer Bestrahlungsplanung). Hierbei wird ein bestimmtes Bestrahlungsmuster rechnerisch simuliert (also ein Ablauf mit unterschiedlichen x-y-Ablenkungen des Teilchenstrahls sowie geeigneten Teilchenenergien des Teilchenstrahls) und der hierdurch jeweils verursachte Dosiseintrag im mit der Strahlung beaufschlagten Körper ortsabhängig berechnet. Denn obgleich die deponierte Dosis im bestrahlten Objekt auf den Bereich des Bragg-Peaks konzentriert ist, wird dennoch (insbesondere in proximal zum Bestrahlungspunkt längs des Teilchenstrahls liegenden Bereichen) eine bestimmte Dosis deponiert. Im Rahmen der Bestrahlungsplanung wird versucht die Teilchenstrahlführung derart zu optimieren, dass innerhalb eines zu therapierenden Gebiets (üblicherweise als CTV = Clinical Target Volume bezeichnet) des Objekts mit einer gewissen Mindestdosis beaufschlagt wird. Umgebendes Material (Gewebe) soll dagegen einer möglichst geringen Dosis ausgesetzt werden.

Besondere Probleme treten auf, wenn sich (Teilbereiche des) zu bestrahlenden Objekts bewegen. Eine Bewegung kann dabei nicht nur Translationsbewegungen, sondern auch Torsionsbewegungen und/oder Stauchungsbewegungen bzw. Dehnungsbewegungen umfassen. Insbesondere in Kombination mit Scanverfahren können dabei die Bewegungen des Objekts und die des Teilchenstrahls miteinander "interferieren" und zu vergleichsweise schlechten Bestrahlungsergebnissen führen, wenn nicht geeignete Gegenmaßnahmen ergriffen werden.

Eine zwischenzeitlich verbreitete Methode um bewegte Zielgebiete bestrahlen zu können, besteht in einer Nachführung des Teilchenstrahls, was fachsprachlich als so genanntes "Tracking" bezeichnet wird. Der Teilchenstrahl wird dabei derart nachgesteuert, dass er die Bewegung des Zielvolumenbereichs im Objekt kompensiert. Bei einer derartigen Strahlnachführung durch Tracking ist es in der Tat möglich, dass der eigentlich zu bestrahlende Rasterpunkt (bzw. die beabsichtigte Strahlposition und/oder der Zielvolumenbereich) im Wesentlichen mit der geplanten Dosis angesteuert werden kann. Weil die Bewegungen im zu bestrahlenden Objekt (insbesondere in Kombination mit den Bewegungen des Teilchenstrahls) in der Planung nicht vorhergesagt werden können, kommt es jedoch zu vorab nicht planbaren Dosiseinträgen in Gebieten, die nicht dem aktuell bestrahlten Rasterpunkt entsprechen. Die im Rahmen eines Objekt-Bearbeitungsvorgangs (bzw. einer Therapiesitzung) eingebrachten Dosen können durch Akkumulation von Dosen, die außerhalb des aktuell angesteuerten Rasterpunkts im Material eingebracht werden, zu einer durchaus signifikanten Abweichung zwischen der Sollplanung und der tatsächlich eingebrachten Dosisverteilung führen.

Es ist daher wünschenswert, dass während der Bestrahlung des Objekts die Bewegung des Objekts gemessen wird, und unter Zuhilfenahme dieser Messungen berechnet wird, wie die tatsächliche Dosisverteilung im bestrahlten Objekt ist. Derartiges Wissen kann beispielsweise noch im Rahmen des Objekt-Bearbeitungsvorgangs verwendet werden (beispielsweise indem die noch folgende Bestrahlung entsprechend angepasst wird), oder aber auch zu einem späteren Zeitpunkt, insbesondere dann, wenn die vollständige Bearbeitung im Rahmen mehrerer, zeitlich voneinander separierter ObjektBearbeitungssitzungen durchgeführt wird.

Verfahren, mit denen eine derartige Dosiseintragskontrolle durchgeführt werden kann, wurden bereits im Stand der Technik vorgeschlagen.

Beispielsweise wurde in der Deutschen Offenlegungsschrift DE 10 2009 055 902 A1 ein Verfahren vorgeschlagen, bei dem in Abhängigkeit von der ermittelten Dosis, welche die i-te Rasterposition bei der Bestrahlung der vorherigen Rasterpositionen bereits erhalten hat, ein Kompensationswert für die i-te Rasterposition berechnet und in Abhängigkeit von dem Kompensationswert für die i-te Rasterposition und von der nominellen Teilchenfluenz für die i-te Rasterposition eine kompensierte Teilchenfluenz für die i-te Rasterposition berechnet wird, um die i-te Rasterposition mit der für die i-te Rasterposition ermittelten kompensierten Teilchenfluenz zu bestrahlen. Die Ermittlung der Differenz zwischen der Solldosis und der Istdosis in der i-ten Rasterposition erfolgt unter Verwendung einer vorausberechneten Datenbasis zur Dosiskompensation in Matrizenform mit einer großen Menge von einzelnen Feldelementen $D_m^{ik}$. Die dort vorgeschlagene Dosiskompensation liefert durchaus brauchbare Ergebnisse. Die Erfahrung hat jedoch gezeigt, dass die Anwendung de facto auf sehr kleinräumige Volumen beschränkt ist, so dass die Anzahl der Rasterpositionen ausreichend klein ist. Für größere Volumen (CTV-Größen jenseits von ca. 10 cm$^3$) steigt jedoch der Speicherbedarf zur Speicherung der Feldelemente übermäßig an. Der Speicherbedarf für die Feldelemente steigt nämlich etwa mit dem Quadrat der Anzahl der Rasterpositionen an. Für Volumina jenseits von etwa 10 cm$^3$ ergeben sich so Speichererfordernisse im Gigabyte-Bereich, was auch mit heutigen Rechnern nur problematisch realisierbar ist. Darüber hinaus steigt auch die benötigte Zeit zur Vorab-Berechnung der Feldelemente $D_m^{ik}$ überproportional an.

In der wissenschaftlichen Veröffentlichung "Treatment Planning for Heavy-Ion Radiotherapy: Physical Beam Model and Dose Optimization" von Krämer M. et al. in Physics in Medicine and Biology, Band 45, Nr. 11 vom 1. November 2000, Seiten 3299-3317, ISSN: 0031-9155/45/11/313 wird ein Computerprogramm zur Planung einer Bestrahlungssitzung mit energetischen Ionen, insbesondere $^{12}$C, beschrieben. Die mit dem Computerprogramm berechnete Bestrahlungsplanung wird anschließend an ein Steuersystem übergeben, welches die Beaufschlagung des Patienten mit dem Teilchenstrahl steuert.

In der internationalen Patentanmeldung WO 2011/153639 A2 wird ein Verfahren zur Berechnung und Optimierung einer Bestrahlungsplanung vorgeschlagen. Bei der Optimierung können unterschiedliche Ziele vorgegeben werden, welche von dem Programm gleichzeitig erzielt werden können.

Die Aufgabe der Erfindung besteht somit darin, ein universeller verwendbares Verfahren zur Ermittlung des Dosiseintrags in außerhalb des aktuell bestrahlten Zielvolumenbereichs liegende Materialbereiche vorzuschlagen. Die Aufgabe besteht weiterhin darin, eine gegenüber dem Stand der Technik verbesserte Vorrichtung zur Ermittlung des Dosiseintrags in außerhalb des aktuell bestrahlten Zielvolumenbereichs liegende Volumenbereiche vorzuschlagen.

Die Erfindung löst die Aufgabe. Die Erfindung ist in den Ansprüchen definiert. Andere Ausführungsbeispiele sind lediglich illustrativ.

Es wird vorgeschlagen, ein Verfahren zur Ermittlung des Dosiseintrags in ein mit einem energetischen Teilchenstrahl zu bestrahlendes Objekts während der Applizierung der Bestrahlung, bei dem zumindest teilweise der Dosiseintrag in Volumenbereiche, welche außerhalb des jeweils vom energetischen Teilchenstrahl bestrahlten Zielvolumenbereichs liegen, ermittelt wird, derart durchzuführen, dass zur Ermittlung des tatsächlich in außerhalb des vom energetischen Teilchenstrahl bestrahlten Zielvolumenbereichs liegende Volumenbereiche eingetragenen Dosiseintrags eine Berechnungsfunktion verwendet wird, welche zumindest teilweise auf einem physikalischen Modell des energetischen Teilchenstrahls beruht. Dabei werden die während der Applizierung der Bestrahlung ermittelten Do-siseinträge lediglich gespeichert und/oder ausgegeben. Weiterhin bewegt sich das zu bestrahlende Objekt während der Applizierung der Bestrahlung zumindest zeitweise und/oder zumindest bereichsweise. Bei dem energetischen Teilchenstrahl handelt es sich bevorzugt um einen dünnen (bleistiftdünnen) Teilchenstrahl, der vorzugsweise während der Applizierung der Bestrahlung bewegt wird. Es kann sich dabei um fachsprachlich als so genannte Scanverfahren bekannte Bewegungsverfahren handeln (insbesondere Raster-Scanverfahren, Spot-Scanverfahren bzw. kontinuierliche Scanverfahren). Der Teilchenstrahl kann insbesondere aus Teilchen bestehen, welche eine (wenn auch kleine) Ruhemasse aufweisen. Insbesondere kann es sich um Hadronen handeln, wie speziell Protonen, Heliumkerne, Ionen, Schwerionen (insbesondere Sauerstoffionen, Kohlenstoffionen, Neonionen), gegebenenfalls auch deren nichtionisierte Äquivalente. Grundsätzlich ist es jedoch auch möglich, dass ein unbewegter Teilchenstrahl verwendet wird und/oder dass der Teilchenstrahl durch geeignete Maßnahmen aufgeweitet wird. Im Übrigen sind auch Leptonen als Teilchen denkbar (insbesondere Elektronen und Positronen). Bei dem Objekt kann es sich um ein grundsätzlich beliebiges Objekt handeln. Insbesondere kann es sich um ein zu bearbeitendes Werkstück, wie beispielsweise ein Halbleitermaterial, ein metallisches Material oder dergleichen handeln. Insbesondere ist es aber auch denkbar, dass es sich um Objekte aus dem medizinischen Bereich handelt. In diesem Zusammenhang ist jedoch nicht nur an menschliche und tierische Patienten zu denken, sondern insbesondere auch an Zellkulturen und so genannte Bestrahlungsphantome, mit denen beispielsweise ein vorab berechneter Bestrahlungsplan verifiziert werden kann, bevor er tatsächlich für eine Therapie genutzt wird. Obgleich Strahlung geladener Teilchen (insbesondere hadronische Teilchenstrahlung) einen üblicherweise ausgeprägten Bragg-Peak aufweist, ist es dennoch nicht zu vermeiden, dass eine gewisse Dosis auch in eigentlich nicht vom Teilchenstrahl "angefahrene" Volumenbereiche eingetragen wird. Dies betrifft insbesondere Volumenbereiche, welche proximal zum aktuell angesteuerten Zielvolumenbereich liegen. Es ist darauf hinzuweisen, dass im Rahmen der vorliegenden Anmeldung unter dem Begriff "Zielvolumenbereich" in der Regel die unmittelbare Umgebung des Bragg-Peaks zu verstehen ist (obgleich zum Teil unter diesem Begriff auch das vom Arzt markierte Zielvolumen - wie zum Beispiel der zu bestrahlende Tumor - verstanden wird). Da die derart eingebrachten Dosen mit der Zeit kumulieren können und insbesondere beim Vorhandensein von Bewegungen des zu bestrahlenden Objekts die hierdurch verursachten Dosiseinträge nicht vorher geplant werden können, ist es sinnvoll, diese Dosiseinträge während der tatsächlichen Applizierung der Bestrahlung zu ermitteln, insbesondere damit diese zu einem späteren Zeitpunkt, der jedoch auch noch während der aktuellen Applikations-Sitzung liegen kann, berücksichtigt werden können. Es ist darauf hinzuweisen, dass diese "unerwünschten Dosiseinträge" einen durchaus signifikanten Anteil am Dosiseintrag in einen bestimmten Volumenbereich ausmachen können und insbesondere durch Bewegungen verursachte Schwankungen zu erheblichen Abweichungen von der im Rahmen der Bestrahlungsplanung berechneten bzw. angenommenen Dosis führen können. Die Erfinder haben festgestellt, dass es bei der Berechnung dieses Dosiseintrags sinnvoll ist, eine Berechnungsfunktion zu verwenden, die zumindest teilweise auf einem physikalischen Modell des energetischen Teilchenstrahls beruht. Zu ihrer eigenen Überraschung war es dadurch möglich, den Speicherbedarf bei größeren zu bestrahlenden Volumenbereichen ganz erheblich zu reduzieren. Insbesondere war es möglich, in einem ersten Versuch mit mehreren zehntausend Rasterpunkten den erforderlichen Speicherbedarf von einem Gigabyte auf knapp unter ein Megabyte zu senken. Darüber hinaus skaliert der Bedarf an Speicher mit dem vorliegend vorgeschlagenen Verfahren nur noch linear (und nicht mehr quadratisch wie beispielsweise bei dem in DE 10 2009 055 902 A1 vorgeschlagenen Verfahren), so dass auch sehr große Objekte sinnvoll bestrahlt werden können. Möglich ist dies vermutlich deshalb, weil bei den bisherigen Verfahren bei der Vorabberechnung der Übertragungskoeffizienten eine (an sich unnötige) große Redundanz zwischen einzelnen Matrizenwerten vorhanden ist. Durch eine Verwendung von physikalischen Modellen können jedoch beispielsweise Symmetrien und dergleichen vorteilhaft ausgenutzt werden, so dass sich große Vorteile, insbesondere hinsichtlich des Speicherbedarfs, ergeben können. Dies führt - wie bereits erwähnt - dazu, dass nunmehr große Objekte de facto erstmalig einer (bewegungskompensierten und/oder adaptiven) Bestrahlung zugänglich gemacht werden können.

Vorgeschlagen wird weiterhin, dass bei dem Verfahren zumindest zeitweise und/oder zumindest teilweise der Dosiseintrag in Volumenbereiche, welche dem vom energetischen Teilchenstrahl bestrahlten Zielvolumenbereich entsprechen, ermittelt wird. Hierdurch wird nicht nur die Kontrolle eines Dosiseintrags in "Abseits vom tatsächlich angesteuerten Rasterpunkt" liegende Volumenbereiche möglich. Vielmehr wird eine Kontrolle der im aktuell angesteuerten Zielvolumenbereich eingetragenen Dosis möglich. Dadurch können auch hier eventuelle Schwankungen und/oder unvorhergesehene Effekte erfasst werden, was in aller Regel zu einer Verbesserung der Bestrahlung bzw. zu einer Verbesserung der Bestrahlungskontrolle führen kann.

Als sinnvoll hat es sich erwiesen, wenn bei dem Verfahren das physikalische Modell des Teilchenstrahls auf einer im Wesentlichen gaußischen Verteilung des Teilchenstrahlprofils beruht. Messungen haben ergeben, dass eine derartige Annahme in aller Regel mit tatsächlichen Teilchenstrahprofilen in guter bis hervorragender Näherung übereinstimmt. Dementsprechend haben erste Versuche ergeben, dass die schlussendlich mit dem vorgeschlagenen Verfahren erzielbaren Ergebnisse besonders gut sind. Der Vollständigkeit halber sollte darauf hingewiesen werden, dass die gaußische Verteilung nicht nur bei (im Wesentlichen) kreisrunden Teilchenstrahprofilen angenommen werden kann, sondern ebenfalls bei beispielsweise ellipsenförmigen bzw. ovalen Teilchenstrahprofilen.

Weiterhin ist es von Vorteil, wenn bei dem Verfahren das physikalische Modell auf einem Energieverlustmodell beim Durchtritt von Materie beruht. Dies entspricht in der Regel den tatsächlichen Gegebenheiten, insbesondere von hadronischen Teilchenstrahlen beim Durchtritt durch Materie. Durch das Modell können auch bei derartigen Teilchensorten die diesen Teilchensorten immanenten Effekte berücksichtigt werden, wie insbesondere der nichtlineare Energieverlust beim Durchtritt durch Materie in Abhängigkeit von der Dichte der Materie und/oder von der (Rest-)Energie der Teilchen (hochgradig nicht-linear, insbesondere im Bereich des Bragg-Peaks). Das Energiemodell kann durch an sich bekannte Integrationsverfahren in eine entsprechende Berechnungsfunktion "überführt" werden.

Da sich das zu bestrahlende Objekt während der Applizierung der Bestrahlung zumindest zeitweise und/oder zumindest bereichsweise bewegt, insbesondere in sich bewegt und bevorzugt zumindest Teilbereiche des zu bestrahlenden Objekts Translationsbewegungen und/oder Rotationsbewegungen und/oder Dehnungs- bzw. Stauchungsbewegungen durchführen, kann das vorgeschlagene Verfahren besonders vorteilhaft verwendet werden. Insbesondere können sich beim Vorhandensein von derartigen Bewegungen besonders große Abweichungen der in außerhalb des aktuell bestrahlten Rasterpunkt liegende Volumenbereiche eingetragene Dosen im Verhältnis zu von einer Soll-Planung ergeben. Insbesondere in Bezug auf Translationsbewegungen und/oder Rotationsbewegungen ist darauf hinzuweisen, dass derartige Bewegungen zu einer Verschiebung von unterschiedlich dichten Materiebereichen in Teilchenstrahrichtung führen können. Dementsprechend kann sich auch die effektive Tiefe des Teilchenstrahls ändern und/oder die Art der Verteilung der deponierten Energie in Materiebereichen längst des Teilchenstrahls hochgradig ändern. Auch bei Dehnungs- bzw. Stauchungsbewegungen kommt es in der Regel zu Dichteveränderungen im zu bestrahlenden Objekt, so dass auch hier besonders starke Effekte bei der Dosisdeposition in Bereichen außerhalb des aktuellen Rasterpunkts resultieren können. Werden diese Dosisdepositionseffekte jedoch insbesondere mithilfe des vorgeschlagenen Verfahrens berücksichtigt, so kann der Therapieerfolg insgesamt in der Regel deutlich verbessert werden.

Besonders vorteilhaft ist es, wenn die Bewegung von zumindest Teilen des zu bestrahlenden Objekts ermittelt wird. Die Bewegung kann dabei beispielsweise durch bildgebende Verfahren (beispielsweise Verfahren unter Verwendung von Röntgenstrahlung, Ultraschall-Verfahren und dergleichen), durch nachzuverfolgende Objekte (beispielsweise implantierte Goldkugeln oder sonstige Markersubstanzen) und/oder durch Bewegungssubstitute ermittelt bzw. zumindest angenähert werden. Ein Bewegungssubstitut ist beispielsweise ein Dehnungsmessstreifen, der um den Brustkorb eines Patienten gelegt wird. Vor der eigentlichen Bestrahlung kann beispielsweise mithilfe von bildgebenden Verfahren eine Korrelation Bewegungszustand - Ausdehnung des Dehnungsmessstreifens erfasst werden. Wird während der eigentlichen Behandlung die Länge des Dehnungsmessstreifens ermittelt, so kann mit guter Näherung im Umkehrschluss auf den aktuellen Bewegungszustand geschlossen werden.

Bei dem vorgeschlagenen Verfahren werden die während der Applizierung der Bestrahlung ermittelten Dosiseinträge gespeichert und/oder ausgegeben. Dadurch ist es insbesondere möglich, dass die gewonnenen Daten archiviert werden können und zu späteren Zeitpunkten wieder verwendet werden können. Dies kann sich nicht nur auf nachfolgende Objektbearbeitungssitzungen beziehen, sondern beispielsweise auch auf Beweissicherungsmaßnahmen, Forschungsvorhaben und dergleichen.

Möglich ist es, wenn bei dem Verfahren das physikalische Modell zumindest zeitweise und/oder zumindest teilweise als analytische Funktion und/oder als Wertetabelle vorliegt. Eine analytische Darstellung kann insbesondere den Speicherbedarf für die Speicherung der Daten nochmals verringern. Dagegen kann sich eine Wertetabelle als vorteilhaft erweisen, wenn die analytische Funktion vergleichsweise komplex ist, so dass eine analytische Berechnung - insbesondere online - zu viel Rechenzeit benötigen würde. Selbstverständlich ist auch ein Kombinationsverfahren denkbar, derart, dass beispielsweise eine analytische Berechnung in x-y-Richtung vorgenommen wird und eine Wertetabelle für die z-Richtung verwendet wird. Auch eine Kombination zwischen beiden Extremen, beispielsweise in dem Sinne, dass eine Wertetabelle interpoliert wird (linear, spline und dergleichen), ist ebenso denkbar und in der Regel auch sinnvoll.

Weiterhin ist es vorteilhaft, wenn der energetische Teilchenstrahl während der Applizierung der Bestrahlung bewegt wird, insbesondere scanartig bewegt wird, bevorzugt rasterscanartig, spotscanartig und/oder kontinuierlich scanartig. Hierdurch ist es auf besonders vorteilhafte Weise möglich, im Wesentlichen beliebig geformte Dosiseintrags-Felder in ein Objekt (einen Objektbereich) einbringen zu können. Das Verfahren kann bei einer derartigen Applizierung besonders vorteilhaft verwendet werden und/oder besonders vorteilhafte Ergebnisse liefern.

Weiterhin wird vorgeschlagen, dass bei dem Verfahren eine Differenz zwischen einer Soll-Dosis und einer tatsächlich applizierten Dosis ermittelt wird. Diese Abweichungen können insbesondere zu einem späteren Zeitpunkt (speziell auch während der aktuellen Dosisapplikation/Bestrahlungsfraktion) berücksichtigt werden. Insbesondere kann ein Dosiseintrag in einen bestimmten Zielvolumenbereich (Rasterpunkt bzw. Strahlposition; zum Teil wird im "Laborjargon" auch - an sich nicht ganz zutreffend - von einem Voxel gesprochen) entsprechend dem derart bestimmten Wert reduziert oder erhöht werden, wenn dieses aktuell vom Teilchenstrahl "angefahren" wird.

Weiterhin wird ein Therapiekontrollsystem mit zumindest einer Kontrolleinrichtung vorgeschlagen, bei dem die Kontrolleinrichtung derart ausgebildet und eingerichtet ist, dass sie ein Verfahren vom vorab beschriebenen Typ aufweist. Das Therapiekontrollsystem kann dann die bereits vorab genannten Eigenschaften und Vorteile in zumindest analoger Weise aufweisen. Darüber hinaus kann das Therapiekontrollsystem im Sinne der vorherigen Beschreibung, zumindest in Analogie, weitergebildet werden.

Besonders vorteilhaft ist es, wenn das Therapiekontrollsystem derart ausgebildet und eingerichtet ist, dass das vom Therapiekontrollsystem ausgeführte Verfahren derart durchgeführt wird, dass der energetische Teilchenstrahl zumindest zeitweise und/oder zumindest bereichsweise die Bewegung des zu bestrahlenden Objekts ausgleicht. Dies kann insbesondere im Sinne von fachsprachlich als so genanntes "Tracking" bezeichneten Nachführungsverfahren aufgefasst werden. Insbesondere ist es möglich, dass die laterale Position des Teilchenstrahls durch eine entsprechende Ablenkung (beispielsweise Ablenkungsmagnet) erfolgt und/oder eine Energieanpassung der Teilchen - und damit eine Anpassung der Eindringtiefe des Teilchenstrahls in das Objekt - erfolgt. Auf diese Weise ist es zumindest möglich, dass der "aktuelle Rasterpunkt" im Wesentlichen mit den Planungen übereinstimmt. Es wird darauf hingewiesen, dass die vorgeschlagene Ausgleichsbewegung sich jedoch auch beispielsweise auf eine Nachführung der Teilchenstrahltrajektorie beziehen kann (so dass dadurch auch ein direkter Einfluss auf einen Strahlungseintrag in Volumenbereiche erfolgen kann, die nicht mit dem aktuell angesteuerten Rasterpunkt-Volumenbereich übereinstimmen). Dies kann durch Bewegung des Teilchenstrahls selbst (beispielsweise durch eine Gantry) erfolgen und/oder beispielsweise durch Bewegung einer Behandlungsliege.

Besonders vorteilhaft ist es, wenn wenn das vorgeschlagene Therapiekontrollsystem derart ausgebildet und eingerichtet ist, dass das vom Therapiekontrollsystem ausgeführte Verfahren derart durchgeführt wird, dass der während der Applizierung der Bestrahlung ermittelte Dosiseintrag einen Einfluss auf die nachfolgende Bestrahlung hat. Auf diese Weise können die ermittelten Werte (insbesondere Abweichungen von vorab angenommenen Werten) "sinnvoll genutzt" werden. Unter einer nachfolgenden Bestrahlung kann einerseits eine Onlineanpassung verstanden werden, so dass bereits eine Anpassung im Rahmen der laufenden Materialbearbeitungssitzung (Therapiesitzung) erfolgen kann. Möglich ist es aber auch, dass es sich um mehrere, zeitlich voneinander beabstandete Materialbearbeitungssitzungen (Therapiesitzungen) handelt, und mithilfe der gewonnenen Daten beispielsweise ein für eine nachfolgende Therapiesitzung zu bestimmender Bestrahlungsplan entsprechend unter Rückgriff auf die so gewonnenen Daten geeignet angepasst werden kann. Eine Anpassung der eingetragenen Dosis kann insbesondere durch eine Veränderung der eingetragenen Teilchenan¬zahl erfolgen. Insbesondere kann eine Veränderung der eingetragenen Teilchenanzahl durch eine Veränderung der Verweildauer eines Teilchenstrahls auf einer bestimmten Strahlposition erfolgen. Eine längere Verweildauer hat üblicherweise einen höheren Teilcheneintrag zufolge (dementsprechend eine kürzere Verweildauer einen geringeren Teilcheneintrag). Zusätzlich oder alternativ ist es aber auch möglich, dass beispielsweise eine Beschleunigervorrichtung entsprechend nachgesteuert wird, derart, dass sich die Teilchenfluenz verändert.

Weiterhin wird ein Bestrahlungssystem vorgeschlagen, welches zumindest ein derartiges Therapiekontrollsystem aufweist. Auch in diesem Fall ist es möglich, dass das Strahlungssystem die bereits vorab vorgeschlagenen Eigenschaften und Vorteile in analoger Weise aufweisen kann und/oder im Sinne der vorherigen Beschreibung zumindest in Analogie weitergebildet werden kann.

Im Folgenden wird die Erfindung anhand vorteilhafter Ausführungsbeispiele und unter Bezugnahme auf die beigefügte Zeichnung näher erläutert. Es zeigen:

Fig. 1:     eine denkbare Vorrichtung zur Applizierung eines energetischen Teilchenstrahls in schematischer Ansicht;
Fig. 2:     eine beispielhafte Skizze zur Erläuterung des Einflusses der Dosisverteilung in proximal zu einem Zielpunkt gelegenen Gewebebereichen, bei Vorhandensein einer Bewegung des Zielobjekts;
Fig. 3:     ein mögliches Beispiel für physikalische Annahmen für ein physikalisches Modell eines energetischen Teilchenstrahls;
Fig. 4     ein mögliches Verfahren zur Ermittlung des Dosiseintrags in ein Objekt in unterschiedlichen Volumenbereichen.

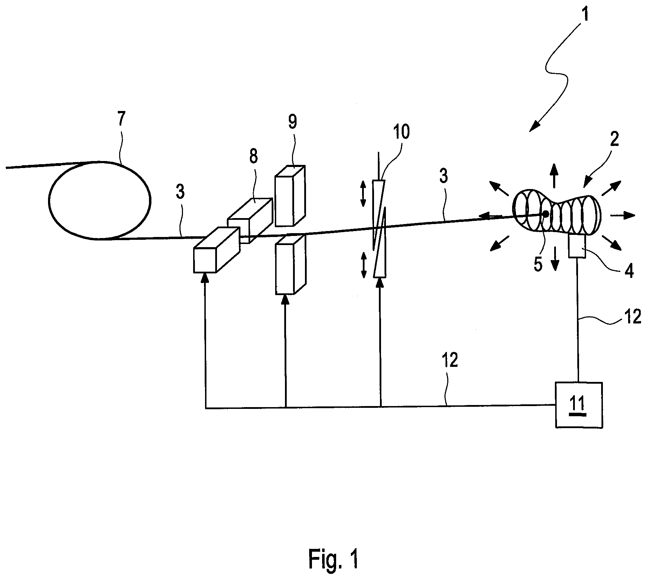

In Fig. 1 ist in schematischer Darstellung eine Bestrahlungsvorrichtung 1 zur Bestrahlung eines Objekts 2 mit einem hochenergetischen Teilchenstrahl 3 (vorliegend Schwerionen) in schematischer Ansicht dargestellt. Die Bestrahlungsvorrichtung 1 ist dabei gleichzeitig derart ausgebildet und eingerichtet, dass eine Bewegung des Objekts 2 (durch Pfeile angedeutet) mithilfe eines Bewegungssubstitut-Messsensors 4 gemessen werden kann (beispielsweise ein Dehnungsmessstreifen, der um den Brustkorb eines Patienten gelegt ist), um auf diese Weise den Dosiseintrag nicht nur in der aktuell bestrahlten Strahlposition 5, sondern auch in anderen Volumenbereichen (Positionen 6), insbesondere in Positionen 14, 15, welche sich proximal zur aktuellen Strahlposition 5 in Teilchenstrahlrichtung 3 gesehen befinden, zu bestimmen. Im "Laborjargon" wird dabei die aktuell bestrahlte Strahlposition 5 zum Teil - nicht ganz zutreffend - als Zielvoxel bezeichnet und dementsprechend die (sonstigen) Positionen 6, 14, 15 als Voxel bezeichnet.

Die Bestrahlungsvorrichtung 1 umfasst einen Beschleuniger, der vorliegend als Synchrotron 7 ausgebildet ist (wobei üblicherweise ein Linearbeschleuniger (Linac) vorgeschaltet ist, der jedoch vorliegend in Fig. 1 aus Übersichtlichkeitsgründen nicht dargestellt ist. Auch die Ionenquelle zur Erzeugung der Ionen im Teilchenstrahl 3 ist in Fig. 1 aus Übersichtlichkeitsgründen nicht dargestellt.

Der hoch beschleunigte Teilchenstrahl 3, der das Synchrotron 7 verlässt, wird durch zwei Ablenkspulenpaare 8, 9 in horizontaler (Ablenkspulenpaar 8) und vertikaler (Ablenkspulenpaar 9) Richtung abgelenkt. Eine (schnelle) Energievariation des Teilchenstrahls 3 ist durch zwei gegeneinander verschiebliche Absorberkeile 10 (Energievariationsvorrichtung) möglich. Mithilfe der Ablenkspulenpaare 8, 9 und der Absorberkeile 10 kann ein im Inneren des zu bestrahlenden Objekts 2 befindliches Zielvolumen durch Anfahren einer großen Anzahl von Strahlpositionen 5 abgescannt, und damit mit einer bestimmten Dosis belegt werden. Gleichzeitig kann mithilfe der Ablenkspulenpaare 8, 9 und der Absorberkeile 10 unter Verwendung der Daten des Bewegungssubstitut-Messsensors 4 ein Tracking (eine Nachverfolgung) der sich bewegenden Strahlposition 5 innerhalb des Objekts 2 durchgeführt werden und auf diese Weise die Bewegung der jeweiligen Strahlposition 5 im Wesentlichen ausgeglichen werden. Die Daten des Bewegungssubstitut-Messsensors 4 werden von einem elektronischen Rechner 11 verarbeitet, der über entsprechende Datenleitungen 12 mit Daten versorgt wird bzw. entsprechende Steuerbefehle an die Ablenkspulenpaare 8, 9 und die Absorberkeile 10 ausgibt.

Selbstverständlich ist es möglich, dass der elektronische Rechner 11 auch noch weitere Daten verarbeitet. Darüber hinaus kann der elektronische Rechner 11 gegebenenfalls auch Daten an das Synchrotron 7 (und/oder andere Bereiche des Beschleunigers) senden, um auf diese Weise beispielsweise größere Energievariationen durchzuführen (insbesondere größere Energievariationen, als diese mit den Absorberkeilen 10 darstellbar sind). Eine Energievariation des Synchrotrons 7 ist jedoch üblicherweise nur von einem Teilchenspill zum anderen durchführbar, benötigt also eine vergleichsweise lange Zeit, so dass die Verwendung von Absorberkeilen 10 (oder einer anderweitigen schnellen Energievariationsvorrichtung) sinnvoll ist.

In den elektronischen Rechner 11 wird vor der Applizierung der Bestrahlung ein vorab berechneter Bestrahlungsplan eingelesen. Da die Bewegungen des Objekts 2 (insbesondere innere Bewegungen des Objekts 2) nicht im Rahmen der Bestrahlungsplanung vorhergesagt werden können, ist eine Messung der Bewegung des Objekts 2 während der eigentlichen Applizierung der Bestrahlung erforderlich, um einerseits Tracking durchführen zu können, andererseits den Dosiseintrag in Volumenbereiche außerhalb der eigentlichen (aktuellen) Strahlposition 5 bestimmen zu können.

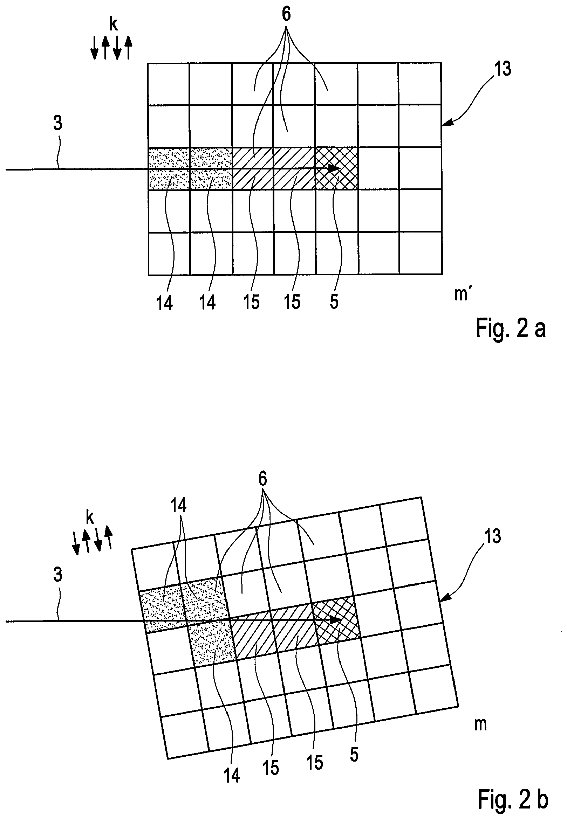

In Fig. 2 sind die Effekte, die durch eine Bewegung eines Objekts 2 im Hinblick auf die Dosisdeposition in unterschiedliche Positionen 6, insbesondere in proximal zur eigentlichen Strahlposition 5 liegende Positionen 14, 15, bewirkt werden, schematisch zur Veranschaulichung dargestellt. In Fig. 2 ist jeweils ein Ausschnitt 13 aus einem zu bestrahlenden Objekt 2 dargestellt. Der Ausschnitt 13 ist in eine Vielzahl von einzelnen finiten, ansteuerbaren Volumenbereichen, vorliegend als Positionen 6 bezeichnet, unterteilt. Weiterhin ist in Fig. 2 der einfallende Teilchenstrahl 3 dargestellt. Da vorliegend der Ausschnitt 13 derart (klein) gewählt ist, dass sämtliche Positionen 6 zu einem gewissen Zeitpunkt vom Teilchenstrahl 3 angefahren werden, sind sämtliche Positionen 6 zu einem bestimmten Bestrahlungszeitpunkt Strahlpositionen 5. Weiter außerhalb des Ausschnitts 13 liegende Positionen 6 werden dagegen (zum Teil) nicht vom Teilchenstrahl 3 angefahren. Dennoch werden auch diese Positionen 6 bei der Ermittlung des jeweils in diese "unbeabsichtigt" eingebrachten Dosiseintrags berücksichtigt. Aufgrund einer Bewegung des Objekts 2 (bzw. Teilen des Objekts 2) bewegt sich der Ausschnitt 13, so dass das Positionsgitter 6 entsprechend verschoben und verdreht wird. Eine einfache Verschiebung (Translationsverschiebung) des Ausschnitts 13 kann durch eine geeignete Ablenkung und Energieanpassung des Teilchenstrahls 3 (entsprechende Ansteuerung der Ablenkspulenpaare 8, 9 und der Absorberkeile 10) in der Regel gut ausgeglichen werden. Eine Rotationsbewegung, wie sie zwischen Fig. 2a (Bewegungszustand m') und Fig. 2b (Bewegungszustand m) auftritt, kann hierdurch jedoch üblicherweise nicht kompensiert werden. Wie man den beiden Teilfigs. von Fig. 2 entnehmen kann, führt eine derartige Verdrehung des Ausschnitts 13 dazu, dass der Teilchenstrahl 3 nunmehr andere Positionen 6 durchdringt, obwohl die gleiche Strahlposition 5 angesteuert wird. Die entsprechend durchdrungenen Positionen 6 (also die proximal zur Strahlposition 5 gelegenen Positionen 14, 15) sind dabei vorliegend zur Veranschaulichung in Positionen 14, in denen eine schwache Energiedeposition stattfindet (punktiert), und Positionen 15, in denen eine mittlere Energiedeposition stattfindet (schraffiert), unterteilt. In der mit einem Kreuzmuster hinterlegten Strahlposition 5 liegt der Hauptbereich des Bragg-Peaks, so dass hier eine sehr starke Energiedeposition stattfindet.

Wie bereits erwähnt, kann der Dosiseintrag in die von der eigentlichen Strahlposition 5 abweichende Positionen 6 (insbesondere in die Positionen 14, 15) erst während der eigentlichen Bestrahlung des Objekts 2 bestimmt werden, da die Bewegungen des Objekts 2 bei der Erstellung der Bestrahlungsplanung nicht bekannt sind. Bislang bediente man sich zur Berechnung der betreffenden Dosisanteile einer Matrix, die eine Abbildungsfunktion von einem Dosiseintrag in eine Strahlposition 5 (wobei sich die Strahlposition 5 im Laufe einer Bestrahlung durch den Scanvorgang ändert) und den sonstigen Positionen 6 beschreibt. Die Koeffizienten dieser Matrix müssen dabei für jede einzelne Bewegungsphase, die realistischerweise im Rahmen der Bestrahlung zu erwarten sind, vorherbestimmt werden. Der Speicherbedarf der entsprechenden Matrix steigt etwa quadratisch mit der Anzahl der berücksichtigten Positionen 6 an. Bereits bei vergleichsweise kleinen Zielgebieten von wenigen Quadratzentimetern ist daher alleine für die Matrix ein Speicherbedarf von mehreren Gigabyte erforderlich. Da es sich um RAM-Speicher handeln muss (ansonsten wäre eine Online-Berechnung nicht möglich, da beispielsweise Festplattenzugriffe zu viel Zeit benötigen), stößt man auch mit heute verfügbaren Rechnern sehr schnell an technische Grenzen.

Dementsprechend wird vorliegend vorgeschlagen, dass zur Berechnung der Dosiseinträge in die unterschiedlichen Positionen 6 anstelle einer mit Koeffizienten gefüllten Matrix eine Berechnungsfunktion verwendet wird, die auf einem physikalischen Modell des Teilchenstrahls beruht.

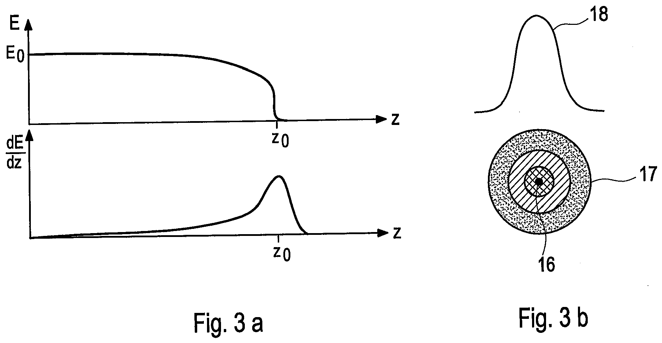

Ein denkbares physikalisches Modell, das zur Erstellung der Berechnungsfunktion genutzt werden kann, ist in Fig. 3 skizziert. Dabei zeigt Fig. 3a das Modell in Ausbreitungsrichtung des Teilchenstrahls 3 (z-Richtung), während Fig. 3b die laterale Erstreckung des Teilchenstrahls darstellt.

In Fig. 3a ist in einem oberen Diagramm die Energie E in Abhängigkeit von der durchdrungenen Materie (Länge z) dargestellt. Dabei wird von einem willkürlich gewählten Anfangsenergiewert $E_0$ ausgegangen. Beim vorliegend als Teilchenstrahl verwendeten Heliumionenstrahl kommt es zunächst zu einem vergleichsweise geringen Energieverlust. Erst kurz vor dem Maximum des Bragg-Peaks bei $z_0$ fängt die Energie an stark abzufallen und erreicht im Bereich des Bragg-Peak-Maximums $z_0$ eine maximale Steigung. Die Verhältnisse sind im unteren Diagramm von Fig. 3a weiter verdeutlicht, wo der differenzielle Energieverlust pro Längeneinheit dE/dz (und damit der Dosiseintrag pro Volumenelement) gegenüber der durchdrungenen Materie in z-Richtung dargestellt ist.

Weiterhin wird in Fig. 3b ein Teilchenstrahl 3 mit einer gaußartigen Intensitätsverteilung um den Mittelpunkt 16 des Teilchenstrahldurchmessers 17 dargestellt. Im oberen Bereich ist eine Gaußkurve 18 dargestellt. Zur Verdeutlichung der Verhältnisse sind um den Mittelpunkt 16 des Teilchenstrahldurchmesser 17 mehrere konzentrische Kreise mit unterschiedlicher Intensität eingezeichnet (wobei in der Realität die Teilchenintensitäten üblicherweise nicht schrittweise, sondern stetig gemäß der Gaußkurve 18 abfallen).

Basierend auf diesem mathematischen Modell erhält man als mögliche Funktion für die Transformationsfunktion $D(E_{beam},r,z)$ die Beziehung:

$$D(E_{beam}, r, z)[\text{Gy}] = 1.6 \cdot 10^{-8} d(E_{beam}, z)\left[\frac{\text{MeV cm}^2}{\text{g}}\right]\frac{N}{2\pi\sigma\sigma^2[\text{mm}^2]}\exp\left(-\frac{r^2}{2\sigma^2}\right) \quad (1)$$

Dabei ist die Funktion $d(E_{beam},z)$ eine Beziehung, die den Dosiseintrag in Abhängigkeit von der Eindringtiefe z beschreibt, und die im Folgenden noch näher erläutert wird. N ist die Teilchenzahl, $\sigma$ ist die Standardabweichung der Teilchenstrahlbreite und r ist der Abstand vom Mittelpunkt 16 des Teilchenstrahldurchmessers 17.

Für die Funktion $d(E_{beam},z)$ kann die Beziehung:

$$d(E_{beam}, z) = \frac{\sum_T \int_E dE \; \frac{dN}{dE}(E_{beam}, z, T, E)(dE)}{\rho dx}(T, E) \quad (2)$$

verwendet werden, die beispielsweise in der wissenschaftlichen Veröffentlichung "Treatment planning for heavy-ion radiotherapy: physical beam model and dose optimization" von M. Krämer, O. Jaeckel, T. Haberer, G. Kraft, D. Schardt und U. Weber in Phy.s Med. Biol. 45 (2000) 3299-3317 hergeleitet wurde, verwendet werden. Dabei ist dN/dE das differenzielle Energiespektrum für eine einzelne differenzielle Isoenergiescheibe. T ist ein Maß für die konkrete Partikelspezies, die durch die Kernladungszahl Z und die Atommasse A definiert wird. $\rho$ steht für die Dichte der durchdrungenen Materie.

Mithilfe der genannten Beziehungen kann daher für jede einzelne bestrahlte Strahlposition 5 (die beispielsweise mit einer bestimmten, fortlaufenden Nummer i bezeichnet ist) der Dosiseintrag in eine andere Position 6 hinein, die beispielsweise mit der fortlaufenden Nummer k versehen ist, in einer Bewegungsphase m berechnet werden. Bildet man die Differenz aus dem derart berechneten, tatsächlichen Strahlungseintrag und dem Strahlungseintrag gemäß Bestrahlungsplanung, so erhält man den Korrekturwert $\Delta d_{m(i)}^{ik}$ aus der Beziehung:

$$\Delta d_{m(i)}^{ik} = D_{norm}\left(E^i, r_m^k, z_m^k\right)\Big|_{x_m^i + \Delta x_m^i} \cdot N_{adapt}^i - D_{norm}\left(E^i, r_{ref}^k, z_{ref}^k\right)\Big|_{x_m^i} \cdot N_{Nom}^i \quad (3)$$

wobei für $D_{norm}\left(E^i, r_m^k, z_m^k\right)$ gilt:

$$D_{norm}(E_{beam}, r, z)[Gy] = 1.6 \cdot 10^{-8} d(E_{beam}, z) \left[\frac{\text{MeV cm}^2}{g}\right] \frac{1}{2\pi\sigma^2[\text{mm}^2]} \exp\left(-\frac{r^2}{2\sigma^2}\right) \quad (4)$$

Bei Formel (4) handelt es sich also um die obige Formel (1), wobei die Teilchenanzahl "N" durch "1" ersetzt ist, mithin also eine (beispielsweise auf eine Teilchenanzahl von "1") normierte Funktion vorliegt.

In Formel (3) wird dann die Skalierung mit der vor der tatsächlichen Bestrahlung unbekannten und in der Regel durch Verwendung der Dosiskompensation selbst sich verändernden Teilchenzahl $N^i_{adapt}$ vorgenommen.

Bei $r^k_m$ handelt es sich im Übrigen um den radialen Abstand der Position k in der Bewegungsphase m relativ zur Strahllage $\vec{x}^i_m$ während der Bestrahlung der Position i in Bewegungsphase m unter Verwendung der Tracking-Parameter $\Delta\vec{x}^i_m$ ("Verschiebung" des Teilchenstrahls 3), während es sich bei $z^k_m$ um die wasseräquivalente Tiefe der Position k in der Bewegungsphase m relativ zur Strahlenlage $\vec{x}^i_m$ während der Bestrahlung der Position i in Bewegungsphase m unter Verwendung der Tracking-Parameter $\Delta\vec{x}^i_m$ handelt.

Der Korrekturwert $\Delta d^{ik}_{m()}$ kann im Rahmen der noch zu applizierenden Bestrahlungsanteile beim Bestrahlen einer entsprechenden Strahlposition 5 berücksichtigt werden. Dies kann beispielsweise durch Veränderung der Verweildauer (kürzer oder länger) des Teilchenstrahls an der betreffenden Strahlposition 5 erfolgen. Ebenso ist es möglich, dass die Daten "lediglich" gespeichert werden und beispielsweise für die Berechnung einer Bestrahlungsplanung bei einer noch zu applizierenden Bestrahlungsfraktion berücksichtigt werden.

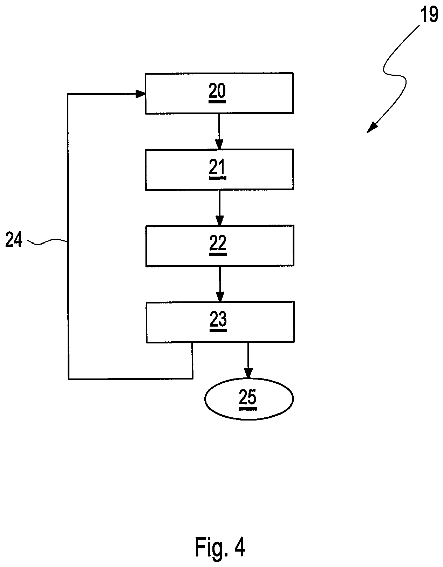

In Fig. 4 ist schließlich noch schematisch ein Flussdiagramm 19 für das vorliegend vorgeschlagene Verfahren dargestellt. In einem ersten Schritt 20 wird die aktuelle Bewegungsphase 20 ermittelt (beispielsweise anhand der Messung eines Bewegungssubstitut-Messsensors 4). Hierauf basierend werden in einem nachfolgenden Schritt 21 die Dosiseinträge in die unterschiedlichen Positionen 6 (insbesondere in die Strahlposition 5 sowie in proximal zu der Strahlposition 5 liegende Positionen 14, 15) berechnet. Hierzu dient eine Funktion, die anhand eines physikalischen Modells ermittelt wurde, wie beispielsweise unter Verwendung der vorab erläuterten Formeln (1) bis (4).

Anschließend wird in einem Schritt 22 die Differenz zwischen geplanter Solldosis und tatsächlich applizierter Dosis 22 ermittelt. Die derart ermittelten Differenzwerte 22 werden in einem Korrekturschritt 23 genutzt, um den Teilcheneintrag in noch zu bestrahlende Zielvoxel 6 noch während der laufenden Bestrahlungsfraktion entsprechend anzupassen (es ist sowohl eine Korrektur nach "oben" sowie nach "unten" möglich).

Anschließend wird an den Anfang des Verfahrens 19 gesprungen 24, solange die Bestrahlung noch nicht vollständig appliziert wurde. Ist die Bestrahlung dagegen vollständig erfolgt, so stoppt das Verfahren (Verzweigung 25). Im Zusammenhang mit diesem Schritt können auch noch die Dosiswerte gespeichert und beispielsweise auf einem Speichermedium ausgegeben werden, so dass diese bei nachfolgenden Bestrahlungsfraktionen in der dazu jeweils zu berechnenden Bestrahlungsplanung berücksichtigt werden können.

Bezugszeichenliste:

1. Bestrahlungsvorrichtung
2. Objekt
3. Teilchenstrahl
4. Bewegungssubstitut-Messsensor
5. Strahlposition
6. Position
7. Synchrotron
8. Ablenkspulenpaar, horizontal
9. Ablenkspulenpaar, vertikal
10. Absorberkeile
11. Elektronischer Rechner
12. Datenleitung
13. Ausschnitt des Objekts
14. Schwache Deposition
15. Mittlere Deposition
16. Mittelpunkt

17. Teilchenstrahldurchmesser
18. Gaußkurve
19. Flussdiagramm
20. Bewegungsphase ermitteln
21. Dosiseinträge ermitteln
22. Differenz bestimmen
23. Korrekturschritt
24. Rücksprung
25. Stopp

**Patentansprüche**

1. Verfahren (19) zur Ermittlung des Dosiseintrags in ein mit einem energetischen Teilchenstrahl (3) zu bestrahlendes Objekt (2), wobei zumindest teilweise der Dosiseintrag in Volumenbereiche (6, 14, 15), welche außerhalb des jeweils vom energetischen Teilchenstrahl (3) bestrahlten Zielvolumenbereichs (5) liegen, ermittelt wird, wobei zur Ermittlung des tatsächlich in außerhalb des vom energetischen Teilchenstrahl (3) bestrahlten Zielvolumenbereichs (5) liegende Volumenbereiche (6, 14, 15) eingetragenen Dosiseintrags eine Berechnungsfunktion verwendet wird, welche zumindest teilweise auf einem physikalischen Modell des energetischen Teilchenstrahls (3) beruht, **dadurch gekennzeichnet, dass**:

   - die Ermittlung des Dosiseintrags während der Applizierung der Bestrahlung durchgeführt wird;
   - sich das zu bestrahlende Objekt (2) während der Applizierung der Bestrahlung zumindest zeitweise und/oder zumindest bereichsweise bewegt und diese Bewegung bei der Ermittlung des Dosiseintrags berücksichtigt wird; wobei die während der Applizierung der Bestrahlung ermittelten Dosiseinträge lediglich gespeichert und/oder ausgegeben werden.

2. Verfahren (19) nach Anspruch 1, **dadurch gekennzeichnet, dass** zumindest zeitweise und/oder zumindest teilweise der Dosiseintrag in Volumenbereichen (5), welche dem vom energetischen Teilchenstrahl (3) bestrahlten Zielvolumenbereich (5) entsprechen, ermittelt wird.

3. Verfahren (19) nach Anspruch 1 oder 2, **dadurch gekennzeichnet, dass** das physikalische Modell auf einem Energieverlustmodell (Fig. 3a) beim Durchtritt durch Materie beruht.

4. Verfahren (19) nach einem der vorangehenden Ansprüche, **dadurch gekennzeichnet, dass** eine Bewegung von zumindest Teilen des zu bestrahlenden Objekts (2) ermittelt wird.

5. Verfahren (19) nach einem der vorangehenden Ansprüche, **dadurch gekennzeichnet, dass** eine Differenz zwischen einer Soll-Dosis und einer tatsächlich applizierten Dosis ermittelt wird.

6. Therapiekontrollsystem (11) mit zumindest einer Kontrolleinrichtung, wobei die Kontrolleinrichtung derartig ausgebildet und eingerichtet ist, dass sie ein Verfahren zur Ermittlung des Dosiseintrags in ein mit einem energetischen Teilchenstrahl (3) bestrahltes Objekt (2) durchführt, wobei zumindest teilweise der Dosiseintrag in Volumenbereiche (6, 14, 15), welche außerhalb des jeweils vom energetischen Teilchenstrahl (3) bestrahlten Zielvolumenbereichs (5) liegen, ermittelt wird, wobei zur Ermittlung des tatsächlich in außerhalb des vom energetischen Teilchenstrahl (3) bestrahlten Zielvolumenbereichs (5) liegende Volumenbereiche (6, 14, 15) eingetragen Dosiseintrags eine Berechnungsfunktion verwendet wird, welche zumindest teilweise auf einem physikalischen Modell des energetischen Teilchenstrahls (3) beruht; **dadurch gekennzeichnet, dass**:

   - die Ermittlung des Dosiseintrags während der Applizierung der Bestrahlung durchgeführt wird;
   - sich das zu bestrahlende Objekt (2) während der Applizierung der Bestrahlung zumindest zeitweise und/oder zumindest bereichsweise bewegt und diese Bewegung bei der Ermittlung des Dosiseintrags berücksichtigt wird.

7. Therapiekontrollsystem (11) nach Anspruch 6, **dadurch gekennzeichnet, dass** zumindest zeitweise und/oder zumindest teilweise der Dosiseintrag in Volumenbereichen (5), welche dem vom energetischen Teilchenstrahl (3) bestrahlten Zielvolumenbereich (5) entsprechen, ermittelt wird.

8. Therapiekontrollsystem (11) nach einem der Ansprüche 6 oder 7, **dadurch gekennzeichnet, dass** das physikalische Modell auf einem Energieverlustmodell (Fig. 3a) beim Durchtritt durch Materie beruht.

9. Therapiekontrollsystem (11) nach einem der Ansprüche 6 bis 8, **dadurch gekennzeichnet, dass** eine Bewegung von zumindest Teilen des zu bestrahlenden Objekts (2) ermittelt wird.

10. Therapiekontrollsystem (11) einem der Ansprüche 6 bis 9, **dadurch gekennzeichnet, dass** der energetische Teilchenstrahl (3) zumindest zeitweise und/oder zumindest bereichsweise die Bewegung des zu bestrahlenden Objekts (2) ausgleicht.

11. Therapiekontrollsystem (11) nach einem der Ansprüche 6 bis 10, **dadurch gekennzeichnet, dass** der während der Applizierung der Bestrahlung ermittelte Dosiseintrag einen Einfluss auf die nachfolgende Bestrahlung hat.

12. Therapiekontrollsystem (11) nach einem der Ansprüche 6 bis 11, **dadurch gekennzeichnet, dass** die während der Applizierung der Bestrahlung ermittelten Dosiseinträge gespeichert und/oder ausgegeben werden.

13. Therapiekontrollsystem (11) nach einem der Ansprüche 6 bis 12, **dadurch gekennzeichnet, dass** der energetische Teilchenstrahl (3) während der Applizierung der Bestrahlung bewegt wird, insbesondere Scanartig bewegt wird, bevorzugt rasterscanartig, spotscanartig und/oder kontinuierlich-scanartig.

14. Therapiekontrollsystem (11) nach einem der Ansprüche 6 bis 13, **dadurch gekennzeichnet, dass** eine Differenz zwischen einer Soll-Dosis und einer tatsächlich applizierten Dosis ermittelt wird.

15. Bestrahlungssystem (1), **gekennzeichnet durch** zumindest ein Therapiekontrollsystem (11) nach einem der Ansprüche 6 bis 14.

**Claims**

1. Method (19) for determining the dose input into an object (2) to be irradiated with an energetic particle beam (3), wherein the dose input is at least partly determined in volume areas (6, 14, 15) located outside the target volume area (5) irradiated by the energetic particle beam (3), wherein to determine the dose input actually input into volume areas (6, 14, 15) located outside the target volume area (5) irradiated by the energetic particle beam (3), a computation function is used which is at least partly based on a physical model of the energetic particle beam (3), **characterized in that**:

   - determination of the dose input is conducted during application of the irradiation;
   - the object (2) to be irradiated moves during application of the irradiation at least at some times and/or at least in some areas and this movement is taken into account when determining the dose input;

   wherein the dose inputs determined during application of the irradiation are only saved and/or output.

2. Method (19) according to claim 1, **characterized in that** the dose input is determined at least at some times and/or at least partly in volume areas (5) corresponding to the target volume area (5) irradiated by the energetic particle beam (3).

3. Method (19) according to claim 1 or 2, **characterized in that** the physical model is based on an energy loss model (Fig. 3a) during passage through material.

4. Method (19) according to one of the preceding claims, **characterized in that** a movement is determined of at least parts of the object (2) to be irradiated.

5. Method (19) according to one of the preceding claims, **characterized in that** a difference is determined between a set dose and an actually applied dose.

6. Therapy control system (11) having at least one control device wherein said control device is designed and equipped such that it performs a method for determining the dose input into an object (2) irradiated with an energetic particle beam (3), wherein the dose input in volume areas (6, 14, 15) located outside the target volume area (5) irradiated by the energetic particle beam (3) is at least partly determined, wherein to determine the dose input actually input into volume areas (6, 14, 15) located outside the target volume area (5) irradiated by the energetic particle beam (3), a computation function is used which is at least partly based on a physical model of the energetic particle beam (3); **characterized in that**:

- determination of the dose input is conducted during application of the irradiation;
- the object (2) to be irradiated moves during application of the irradiation at least at some times and/or at least in some areas and this movement is taken into account when determining the dose input.

7. Therapy control system (11) according to claim 6, **characterized in that** the dose input is determined at least at some times and/or at least partly in volume areas (5) corresponding to the target volume area (5) irradiated by the energetic particle beam (3).

8. Therapy control system (11) according to one of claims 6 or 7, **characterized in that** the physical model is based on an energy loss model (Fig. 3a) during passage through material.

9. Therapy control system (11) according to one of claims 6 to 8, **characterized in that** a movement is determined of at least parts of the object (2) to be irradiated.

10. Therapy control system (11) according to one of claims 6 to 9, **characterized in that** the energetic particle beam (3) compensates at least at some times and/or at least in some areas for the movement of the object (2) to be irradiated.

11. Therapy control system (11) according to one of claims 6 to 10, **characterized in that** the dose input determined during application of the irradiation has an effect on the subsequent irradiation.

12. Therapy control system (11) according to one of claims 6 to 11, **characterized in that** the dose inputs determined during application of the irradiation are saved and/or output.

13. Therapy control system (11) according to one of claims 6 to 12, **characterized in that** the energetic particle beam (3) is moved during application of the irradiation, in particular in a scanning motion, preferably raster scanning, spot scanning and/or continuous scanning.

14. Therapy control system (11) according to one of claims 6 to 13, **characterized in that** a difference is determined between a set dose and an actually applied dose.

15. Irradiation system (1), **characterized by** at least one therapy control system (11) according to one of claims 6 to 14.

**Revendications**

1. Procédé (19) permettant de déterminer la dose introduite dans un objet (2) à être irradié par un faisceau de particules énergétiques (3), sachant qu'est déterminée au moins partiellement la dose introduite dans des zones volumiques (6, 14, 15) qui se trouvent respectivement hors de chaque zone volumique cible (5) irradiée par le faisceau de particules énergétiques (3), et que pour déterminer la dose effectivement introduite dans des zones volumiques (6, 14, 15) se trouvant hors de la zone volumique cible (5) irradiée par le faisceau de particules énergétiques (3) est utilisée une fonction de calcul qui repose au moins partiellement sur un modèle physique du faisceau de particules énergétiques (3),
**caractérisé en ce que** :

- la détermination de la dose introduite est effectuée pendant l'application de l'irradiation ;
- pendant l'application de l'irradiation, l'objet (2) à être irradié se déplace au moins par intermittence et/ou au moins par zones et que ce mouvement est pris en compte lors de la détermination de la dose introduite ;

les doses introduites déterminées pendant l'application de l'irradiation étant uniquement enregistrées et/ou sorties.

2. Procédé (19) selon la revendication 1, **caractérisé en ce qu'**au moins par intermittence et/ou au moins partiellement est déterminée la dose introduite dans des zones volumiques (5) qui correspondent à la zone volumique cible (5) irradiée par le faisceau de particules énergétiques (3).

**3.** Procédé (19) selon la revendication 1 ou 2, **caractérisé en ce que** le modèle physique repose sur un modèle de perte d'énergie (Fig. 3a) lors du passage au travers de matière.

**4.** Procédé (19) selon l'une des revendications précédentes, **caractérisé en ce qu'**est déterminé un mouvement au moins de parties de l'objet (2) à être irradié.

**5.** Procédé (19) selon l'une des revendications précédentes, **caractérisé en ce qu'**est déterminée une différence entre la dose théorique et une dose effectivement appliquée.

**6.** Système de contrôle de thérapie (11) comprenant au moins un dispositif de contrôle, sachant que le dispositif de contrôle est conçu et ajusté de manière telle qu'il réalise un procédé pour déterminer la dose introduite dans un objet (2) irradié par un faisceau de particules énergétiques (3), qu'est déterminée au moins partiellement la dose introduite dans des zones volumiques (6, 14, 15) qui se trouvent hors de chaque zone volumique cible (5) irradiée par le faisceau de particules énergétiques (3), que pour déterminer la dose effectivement introduite dans des zones volumiques (6, 14, 15) se trouvant hors de la zone volumique cible (5) irradiée par le faisceau de particules énergétiques (3) est utilisée une fonction de calcul qui repose au moins partiellement sur un modèle physique du faisceau de particules énergétiques (3) ;

**caractérisé en ce que**:

- la détermination de la dose introduite est effectuée pendant l'application de l'irradiation ;
- pendant l'application de l'irradiation, l'objet (2) à être irradié se déplace au moins par intermittence et/ou au moins par zones et que ce mouvement est pris en compte lors de la détermination de la dose introduite.

**7.** Système de contrôle de thérapie (11) selon la revendication 6, **caractérisé en ce qu'**au moins par intermittence et/ou au moins partiellement est déterminée la dose introduite dans des zones volumiques (5) qui correspondent à la zone volumique cible (5) irradiée par le faisceau de particules énergétiques (3).

**8.** Système de contrôle de thérapie (11) selon l'une des revendications 6 ou 7, **caractérisé en ce que** le modèle physique repose sur un modèle de perte d'énergie (Fig. 3a) lors du passage au travers de matière.

**9.** Système de contrôle de thérapie (11) selon l'une des revendications 6 à 8, **caractérisé en ce qu'**est déterminé un mouvement au moins de parties de l'objet (2) à être irradié.

**10.** Système de contrôle de thérapie (11) selon l'une des revendications 6 à 9, **caractérisé en ce que** le faisceau de particules énergétiques (3) compense au moins par intermittence et/ou au moins par zones le mouvement de l'objet (2) à être irradié.

**11.** Système de contrôle de thérapie (11) selon l'une des revendications 6 à 10, **caractérisé en ce que** la dose introduite déterminée pendant l'application de l'irradiation a une influence sur l'irradiation suivante.

**12.** Système de contrôle de thérapie (11) selon l'une des revendications 6 à 11, **caractérisé en ce que** les doses introduites déterminées pendant l'application de l'irradiation sont enregistrées et/ou sorties.

**13.** Système de contrôle de thérapie (11) selon l'une des revendications 6 à 12, **caractérisé en ce que** pendant l'application de l'irradiation, le faisceau de particules énergétiques (3) est mis en mouvement, en particulier selon un mouvement de balayage, de préférence selon un mouvement de balayage tramé, de balayage ponctuel et/ou de balayage continu.

**14.** Système de contrôle de thérapie (11) selon l'une des revendications 6 à 13, **caractérisé en ce qu'**est déterminée une différence entre une dose théorique et une dose effectivement appliquée.

**15.** Système d'irradiation (1) **caractérisé par** au moins un système de contrôle de thérapie (11) selon l'une des revendications 6 à 14.

Fig. 1

Fig. 2 a

Fig. 2 b

Fig. 3 a

Fig. 3 b

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102009055902 A1

- WO 2011153639 A2

**In der Beschreibung aufgeführte Nicht-Patentliteratur**

- **KRÄMER M. et al.** Treatment Planning for Heavy-Ion Radiotherapy: Physical Beam Model and Dose Optimization. *Physics in Medicine and Biology,* 01. November 2000, vol. 45 (11), ISSN 0031-9155/45/11/313, 3299-3317

- **M. KRÄMER ; O. JAECKEL ; T. HABERER ; G. KRAFT ; D. SCHARDT ; U. WEBER.** Treatment planning for heavy-ion radiotherapy: physical beam model and dose optimization. *Phy.s Med. Biol.,* 2000, vol. 45, 3299-3317